# EUROPEAN PATENT APPLICATION

(11) **EP 2 805 730 A1**
(43) Date of publication of application: **26.11.2014**
(21) Application number: 13168487.0
(22) Date of filing: 21.05.2013
(51) Int. Cl.: A61K 39/395, A61K 45/06, A61K 31/00, A61K 31/198, A61K 31/21, A61K 31/34, A61K 31/519, A61P 25/00

(54) **Nitric oxide donor for the treatment of chronic fatigue syndrome**

(71) Applicant: Bergen Teknologioverføring AS, 5006 Bergen (NO)
(72) Inventor: Fluge, Oystein, 5122 Morvik (NO); Mella, Olav, 5184 Olsvik (NO)
(74) Representative: Kröncke, Rolf

(57) **Abstract**

The present invention relates in a first aspect to a nitric oxide donor for use in the treatment of chronic fatigue syndrome also known as myalgic encyphalomyelitis. In particular, the present invention relates to the use of a pharmaceutical composition containing a nitric oxide donor for the treatment of chronic fatigue syndrome/myalgic encyphalomyelitis in a subject afflicted with said disease.

In a further aspect, the present invention relates to a combination of a nitric oxide donor with a B-cell depleting agent for use in the treatment of chronic fatigue syndrome. Said combination may be provided in form of a kit comprising pharmaceutically effective dosages of said compounds. Further, the present invention relates to a method for treating chronic fatigue syndrome comprising the step of administering nitric oxide donor to a subject afflicted therewith.

## Description

The present invention relates in a first aspect to a nitric oxide donor for use in the treatment of chronic fatigue syndrome also known as myalgic encyphalomyelitis. In particular, the present invention relates to the use of a pharmaceutical composition containing a nitric oxide donor for the treatment of chronic fatigue syndrome/myalgic encyphalomyelitis in a subject afflicted with said disease. In a further aspect, the present invention relates to a combination of a nitric oxide donor with a B-cell depleting agent for use in the treatment of chronic fatigue syndrome. Said combination may be provided in form of a kit comprising pharmaceutically effective dosages of said compounds. Further, the present invention relates to a method for treating chronic fatigue syndrome comprising the step of administering a nitric oxide donor to a subject afflicted therewith.

### Technical background

Chronic fatigue syndrome (CFS) also described as myalgic encyophalitis (ME) is a disease affecting approximately 0.2 percent of the population (Nacul et al, BMC med 2011, 9:91). It is a disease affecting women three to four times more often than men and often preceded by an infection. It is speculated on a genetic predisposition for CFS (Albright et al, 2011, BMC neurol 11 :62). According to the clinical working case definition (Canadian criteria) for CFS/ME, Carruthers B.M., et al., 2003, J. Chronic Fatigue Syndr, 11 :7-36, the main symptoms are post-exertional malaise, with cognitive disturbances, pain, sensory hypersensitivity, and several symptoms related to neuroendocrine and autonomic function. CFS is characterized by an unexplained, severe fatigue, persisting for at least six consecutive months, and with a substantial reduction of previous levels in occupational, social or personal activities. Although many studies have been shown subtle alterations in blood tests or radiological investigations, no biomarker or diagnostic test exist.

That is, the aethiology of CFS remains unclear. Various hypotheses include immunological, virological, neuroendocrinological, and psychological mechanisms. The pathogenesis of CFS is presumed to be multifactorial and to involve both host and environmental factors.

Many patients suffering from CFS have a history of an acute viral infection preceding the development of fatigue. However, no triggering viral infection has been proven yet.

Furthermore, several gene expression studies have been performed in CFS, indicating that there are specific but complex gene alterations in accordance with the dysfunction in immune response and in defence mechanisms. For example, Kaushik N., et al., 2005, J. Clin Pathol 58:826-32 describe a microarray study showing differential expression of 16 genes in CFS suggesting T-cell activation and a disturbance of neuronal and mitochondrial function. Other microarray studies concluded that several genes affected mitochondrial function and cell cycle deregulation. Moreover, alterations in membrane transport and ion channels were described. Based on the numerous studies, the gene expression data are not conclusive but suggest that there are disturbances in CFS representing various cellular functions.

A study in 2007 of post-infective fatigue syndrome found no differences in ex vivo cytokine production over a 12-month period, as compared to controls recovering promptly after infection (Vollmer-Conna U., et al., 2007, Clin Infect Dis 45:732-5). It is speculated that CFS patients may have a reduced immune cell function with a low NK cell cytotoxicity and immunoglobulin deficiencies.

For example, Ogava M., et al., 1998, Eur J of Clin Invest, 28:937-943 describes decreased nitric oxide mediated natural killer cell activation in chronic fatigue syndrome. It is identified therein, that NO donor were able to stimulate NK cell activity in healthy control subjects but not in NK cells obtained from CFS patients and stimulated in vitro.

Studies demonstrated several abnormalities in laboratory markers associated with immune functions in CFS patients. For example, a low NK cell cytotoxicity, but also an increase in CD8 + T cells, elevated numbers of CD20+ B-cells, and an increase in the B-cell subset expressing CD20 and CD5 has been described. A recent study comparing CFS patients and controls, reported decreased expression of CD69 on T cells in NK cells after mitogenic stimulation in vitro, indicating a disorder in the early activation of cellular immunity mediated by the cells (Mihaylova I., et al., 2007, Neuro Endocrinol Lett 28:477-83).

However, the data on immune bioregulation in CFS are not consistent, e.g. as discussed in Brenu et al, 2012, J Trans Med 10:88.

Along with hypotheses of immune deregulation in CFS, autoimmunity to endogenous vasoactive non peptides has been proposed as a mechanism for the disease, Staines DR., 2005, Med Hypotheses 64:539-42, however not supported by scientific data. Other reports are discussing various autoantibodies in conjunction with CFS. However, no clear association was proved. Thus, there is no direct evidence with consistent data for the presence of pathogenic autoantibodies or for T-lymphocyte mediated autoimmunity. That is, CFS is at present not defined as an autoimmune disease. Rather, CFS is still identified as a disease with unknown aethiology.

Various hypotheses for CFS pathogenesis are discussed in the art including blood platelet dysfunction, neurological, neuroendocrine, metabolic or autonomic disturbances, ion channel dysfunction, zinc deficiency, toxin exposure or prior vaccination, etc., However, no consistent picture has emerged for the aetiology and pathogenesis of CFS.

Due to the lack of knowledge of the exact pathogenesis and with no known causal mechanism, there is no current standard specific treatment for CFS.

The unknown aethiology of CFS is probably the reason for the remarkably few studies performed, evaluating therapy based upon a biological hypothesis.

Studies are described in the art testing treatment with immunoglobuline, treatment with compounds (ethanercept) to inhibit the TNF mediated cellular response or anti-viral compositions, like valganciclovir.

The inventors of the present invention published a Case series, Fluge O., Mella O., 2009, BMC Neurol. 9:28 followed by a double-blinded and placebo controlled, randomized phase II study, Fluge O., et al., 2011, PLOS 6:e26359, exploring B-cell depletion using the therapeutic monoclonal anti-CD20 antibody Rituximab, showing a clinical benefit in 2/3 of CFS patients. The use of B-cell depleting agents is described in WO 2009/083602. The patterns of responses and relapses, with a time delay of 2 to 8 eight months from start of Rituximab infusion (with rapid B-cell depletion) until start of clinical responses, indicate that an antibody may be involved in the pathogenesis. Recently, a case control study performed in elderly aged more than 65 years, investigating more than 1 million cases in cancer and 100.000 healthy controls, with a prevalence of CFS diagnosis of 0.5 percent in both groups, show that elderly CFS patients had a modest but highly significant risk of B-cell lymphomas, Chang C.M., Cancer, 2012, 118:5929-36, consistent with a chronic B-cell activation. Taken together, the data on treatment with a monoclonal anti-CD20 antibody examplified by Rituximab indicate that CFS in a subset of patients may be a post-infectious immune dysregulation, possibly a variant of autoimmune mechanisms, possibly with a genetic predisposition, in which B lymphocytes are important for symptom maintenance.

However, as indicated above, there is a significant delay from start of the treatment and the beginning of the symptoms relief. This is not only true for treatment with Rituximab, but can also be seen with treatment of methotrexate, a small molecule known as an active agent for B-cell depletion useful in the treatment of various kinds of diseases.

Thus, there is an ongoing need to provide additional compounds useful in the treatment of chronic fatigue syndrome. In particular, there is an ongoing need for providing compounds which act fast in the patients without the lag period described for the B-cell depleting agent. In addition, there is a continuous demand for compounds which may also be effective in patients not susceptible to B-cell depleting agent treatment.

### Brief description of the present invention

In a first aspect, the present invention relates to the use of a nitric oxide donor (NO donor) for use in the treatment of chronic fatigue syndrome (CFS).

That is, the present inventors recognized that administration of a NO donor relieve the symptoms of CFS and, thus, may be useful in the treatment of chronic fatigue syndrome.

In particular, the present inventors recognized that an immediate relief after administration of an NO donor can be observed. In contrast to medication administered so far for treatment of CFS, which are characterized by a remarkable lag time of treatment success, as described for example for the treatment with Rituximab or Methotrexate, see e.g. WO 2009/083602. Thus, the administration of a NO donor surprisingly allow a treatment of CFS patients for immediate relief of symptoms without any delay as described for e.g. a B-cell depleting agent, like Rituximab, before. An alternative strategy was applied, using supplement with relatively high doses of L-Arginine 5 g twice daily combined with L-Citrulline 200 mg twice daily. L-Arginine is the sole substrate of Nitric Oxide Synthases (NOS), thus this approach aims to overcome the dysregulated nitric oxide system in CFS/ME patients by providing relatively high doses of the substrate.

Further, the skilled person is well aware of other compounds suitable to influence the NO level, e.g. by influencing the amount or level of available substrate for NO production. Examples thereof include compounds inhibiting arginase, e.g. arginase II, a competitor of the NO synthase (NOS). Other examples comprise compounds stimulating the activity of NO synthase, in particular, of the eNOS, either by increasing the amount or level of NOS or the turn over rate of the enzyme. These compounds may act directly or indirectly through affecting the level of natural eNOS stimulators, like sphinogine 1-phospate.

Hence, in another aspect, the present invention relates to a pharmaceutical composition comprising a NO donor optionally in combination with a pharmaceutically acceptable diluents, excipient or carrier for use in the treatment a chronic fatigue syndrome.

Moreover a composition is provided containing a combination of a nitric oxide donor and a B-cell depleting agent. Said composition is particular useful in form of a pharmaceutical composition for use in the treatment of chronic fatigue syndrome. That is, while an immediate relief is obtained with the first component, the NO donor, a delayed response is obtained by the treatment with the B-cell depleting agent, as described in the art. Hence, it is desired to administer the pharmaceutical composition containing the combination of NO donor and B-cell depleting agent in a combination, either simultaneously, separately, or sequentially.

Furthermore, the present invention provides a kit comprising as a first component a NO donor and as a second component a B-cell depleting agent. Said kit is useful in the treatment of chronic fatigue syndrome.

Finally, the present invention provides methods for treating chronic fatigue syndrome comprising the step of administering daily a nitric oxide donor. Preferably, said method of treatment includes the combinatorial treatment with a B-cell depleting agent.

### Detailed description of the present invention

The present invention concerns a nitric oxide donor (NO donor) for use in the treatment of a chronic fatigue syndrome (CFS).

In the context of the present invention, the terms "chronic fatigue syndrome", CFS, and "Myalgic Encephalitis", are used synonymously.

As used herein, the term nitric oxide donor (NO donor) refers to compounds which after non-enzymatic or enzymatic reaction release nitric oxide (NO). It is noted that the term NO donor "include NO" unless otherwise specified. However, typically, a NO donor compound is a compound that releases NO or a related redox species and more generally provides nitric oxide bioactivity, e.g. vasorelaxation or stimulation or inhibition of a receptor protein. That is, the term NO donor includes other compounds suitable to influence the NO level, e.g. by influencing the amount or level of available substrate for NO production. The skilled person is well aware of suitable compounds. Examples thereof include compounds inhibiting arginase, e.g. arginase II, a competitor of the NO synthase (NOS). Other examples comprise compounds stimulating the activity of NO synthase, in parituclar, of the eNOS, either by increasing the amount or level of NOS or the turn over rate of the enzyme. These compounds may act directly or indirectly through affecting the level of natural eNOS stimulators, like sphinogine 1-phospate.

Compounds that contain S-nitroso groups, O-nitroso-groups, and N-nitroso groups are all known to release nitric oxide. O-nitroso compounds are compounds having one more -O-NO groups, and are also referred to as O-nitrosylated compounds and nitrite compounds. S-nitroso compounds are compounds with one or more -S-NO groups and are also referred to as nitrosothiols and S-nitrosylated compounds. An -S-NO group is also referred to in the art as a sulfonyl nitrite, a thionitrous acid ester, an S-nitrosothial or a thionitrite. Compounds having an =N-NO group are referred to herein as N-nitroso compounds. Other NO compound includes NONOates, nitroprusside, nitrates, furoxans, etc.

In addition, nitro compounds -Y-NO₂ are included in the embodiment (where Y is N, C, O, S or transition metal).

The NO donor compound can also be an organic nitrite or nitrate selected from the group consisting of amyl nitrate, ethyl nitrite, ethyl nitrate, isosorbide mononitrate, isosorbide dinitrate, nitroglycerin, nitrosothiols and nitroprussides.

For examples, nitric oxide donors for use according to the present invention include without limitation, isosorbide dinitrate, L-arginine, linsidomine, minoxidil, nicorandil, nitroglycerin, nitroprusside, nitrosoglulthathione, and S-nitroso-N-acetyl-penicillamine (SNAP) or glyceryl trinitrate. It is preferred, that isosorbide mononitrate or glyceryl trinitrate as well as L-arginine is used as organic NO donor.

Further, the NO donor includes at least one organic nitrate which includes esters of nitric acid and may be an acyclic or cyclic compound, such as represented by the following general formula:

R(-CR'R"-O-NO₂)ₓ

wherein:
- R is an organic or H (hydro) moiety or covalent bond, preferably a 2 to about 12 carbon hydrocarbon or oxygen-substituted hydrocarbon, especially one having 2 to 6 carbons and from 0 to 2 oxygen(s);
- R' is an organic or hydro moiety or covalent bond, and preferably methyl;
   lower alkyl, to include ethyl, propyl, butyl, pentyl, and hexyl; methoxy; lower alkoxy; or hydro;
- R" is an organic or hydro moiety or covalent bond, preferably methyl, lower alkyl, methoxy, lower alkoxy, or hydro, and especially hydro; and
- X is an integer from 1 to about 12, and preferably from 2 to 6.

For instance, the organic nitrate may be ethylene glycol dinitrate, isopropyl nitrate; glyceryl-1-mononitrate; glyceryl-1 ,2-dinitrate; glyceryl-1,3-dinitrate; nitroglycerin (GTN); butane-1,2,4-triol-trinitrate; erythrityl tetranitrate (ETN); pentaerythrityl tetranitrate (PETN); isosorbide mononitrate (ISMN), which may include isosorbide-2-monotritate (IS2N) and/or isosorbide-5-mononitrate (IS5N); and/or isosorbide dininitrate (ISDN), and so forth and the like. An advantageous organic nitrate is GTN, and advantageous, other organic nitrates include ISDN, ETN, PETN, etc., which may have been given regulatory approval for use in treatments in other fields of medicine on human subjects.

In another preferred embodiment, the nitric oxide donor is a small molecule.

It is particular preferred, that the nitric oxide donor is a compound selected from an organic nitrite, an organic nitrate, a nitrite ester of a polyol, a nitrate ester of a polyol molsidomine and its metabolites, a diazeniumdiolate, a S-nitrosothiol, an iron-sulphur nitrosyl, sodium nitrite, ethylene glycol dinitrate, isopropyl nitrate, amyl nitrite, amyl nitrate, ethyl nitrite, butyl nitrite, isobutyl nitrite, octyl nitrite, glyceryl-1-monoitrate, glyceryl-1,2-dinitrate, glyceryl-1,3-dinitrate, nitroglycerin, butane-1,2,4-triol-trinitrate, erythrityl tetranitrate, pentaerythrityl tetranitrate, sodium nitroprusside, clonitrate, erythrityl tetranitrate, isosorbide mononitrate, isosorbide dinitrate, mannitol hexanitrate, mesoionic oxatriazole, pentaerythritol tetranitrate, penetrinitol, triethanolamine trinitrate, trolnitrate phosphate (triethanolamine trinitrate diphosphate), propatylnitrate, nitrite esters of sugars, nitrate esters of sugars, sodium nitroprusside, nicorandil, apresoline, diazoxide, hydralazine, hydrochlorothiazide, minoxidil, pentaerythritol, tolazoline, scoparone (6,7-dimethoxycoumarin) sinitrodil, sildenafil, vardenafil, tadalafil, 4-Ethyl-2-[(Z)-hydroxyiminol]-5-nitro-3(E)-hexeneamide, L-arginine and pharmaceutically acceptable salts, derivatives and isomers thereof.

The route of administration of the NO donor depends on the formulation used. That is, the NO donor may be administered in form of capsules, e.g.in case of isosorbide mononitrate as well as in form of a plaster (e.g. in case of using Glyceryl trinitrate as NO donor). In addition, the NO donor may be in form of a component of immediate release of NO or in form of a delayed or sustained release of NO. Further, L-Arginine and/or L-Citrulline may be provided in powder form for oral use.

For example, the NO donor is adapted for systemic administration, for example via the enteral or parenteral route. In another embodiment, the NO donor is adapted for mucosal or local administration.

Moreover, in another embodiment, the nitric oxide donor for use according to the present invention is adapted for the administration to a subject in a single therapeutically effective doses or multiple of therapeutically effective doses thereof of. The skilled person is well aware of the effective doses to be administered. Typically, the daily dosage is similar to the daily dosage administered in the treatment of other disease treated with said NO donor, e.g. in case of Imdur®, 30-120 mg/day as example.

Typically, the nitric oxide donor is for use in the treatment of CFS is in a suitable pharmaceutical form, for example in combination with a pharmaceutically acceptable diluent, excipient or carrier. The pharmaceutical composition may contain additional components including pharmaceutical additives, pH-stabilizer, etc.

That is, the present invention provides a pharmaceutical composition comprising the NO donor as defined herein and a pharmaceutically acceptable diluent, excipent or carrier for use in the treatment of CFS.

As used herein, the term "comprising", "comprises", "containing" or "contains" includes the embodiments of "consisting of" or "consist".

Another embodiment of the present invention relates to a composition containing a combination of a NO donor as defined herein and a B-cell depleting agent. Said composition is particularly useful as a pharmaceutical composition, e.g. for use in the treatment of chronic fatigue syndrome.

That is, it is preferred that the pharmaceutical composition is a composition containing a combination of a nitric oxide donor and a B-cell depleting agent for use in the treatment of chronic fatigue syndrome wherein the combination is administered simultaneously, separately or sequentially.

In a preferred embodiment, the pharmaceutical composition is designed to allow administration of the NO donor in a pharmaceutically effective dosage over a time range of the first six weeks of treatment, preferably over a time range of the first eight weeks of treatment, like within three month or four month from the beginning of the treatment. Of course, the treatment regimen depends on the drug administered as well as on the way of administration. The skilled artisan is well aware of suitable dosages and treatment regimen depending on the drug. For example, the same dosages of the NO donor drugs may be administered as it is the case for other types of diseases or disorders the same NO donors are useful.

In addition, the pharmaceutical composition is designed that the B-cell depleting agent is adapted for administration 1 or 2 in fusions twice within the first two weeks and, there after, administering the B-cell depleting agent once every two or three month for maintaining the beneficial effect.

As used herein, the term "B-cell depletion" or "B-cell depleting activity" refers to the ability of the entity, either a chemical or biological entity, e.g. an antibody, to reduce circulating B-cell levels in a subject. B-cell depletion may be achieved e.g. by inducing cell death or reducing proliferation.

The "CD20" antigen, or "CD20," is an about 35-kDa, non-glycosylated phosphoprotein found on the surface of greater than 90% of B cells from peripheral blood or lymphoid organs in humans. CD20 is present on both normal B cells as well as malignant B cells, but is not expressed on stem cells. Other names for CD20 in the literature include "B-lymphocyte- restricted antigen" and "Bp35". The CD20 antigen is described in Clark et al. Proc. Natl. Acad. Sd. (USA) 82:1766 (1985), for example. The term CD20 includes the equivalent molecules of other species than human. Recently, low level expression of CD20 on a subset of T-cells and NK-cells has been reported.

A "B-cell" is a lymphocyte that matures within the bone marrow, and includes a naive B cell, memory B cell, or effector B cell (plasma cells).

In a broader sense, the present invention relates not only to the use of antibodies or fragments thereof for the treatment of CFS but to the use of antagonists of the CD20 molecule in general having a B-cell depleting activity for the treatment of CFS.

An "antagonist" or "B-cell depleting agent" which is used herein interchangeably is a molecule which, e. g. upon binding to a B cell surface marker, like CD20 on B cells, destroys or depletes B cells in a mammal and/or interferes with one or more B cell functions, e.g. by reducing or preventing a humoral response elicited by the B cell. The antagonist or B-cell depleting agent according to the present invention is able to deplete B cells (i.e. reduce circulating B cell levels) in a mammal treated therewith. Such depletion may be achieved via various mechanisms such antibody-dependent cell-mediated cytotoxicity (ADCC) and/or complement dependent cytotoxicity (CDC), inhibition of B cell proliferation and/or induction of B cell death (e.g. via apoptosis). Antagonists included within the scope of the present invention include antibodies, synthetic or native sequence peptides and small molecule antagonists which bind to the B cell surface marker, optionally conjugated with or fused to a cytotoxic agent. A preferred antagonist is a CD20 antibody or CD20-binding antibody fragment. Furthermore, small molecule antagonists are preferred, like the known B-cell depleting agent Methotrexat.

Insofar that other cells than B-cells express the CD20 antigen like a subset of T-cells or NK-cells, these cells are also depleted with the B-cells depleting agent being an agent acting via CD20.

Antagonists which "induce apoptosis" are those which induce programmed cell death, e.g. of a B cell, as determined by standard apoptosis assays, such as binding of annexin V, fragmentation of DNA, cell shrinkage, dilation of endoplasmic reticulum, cell fragmentation, and/or formation of membrane vesicles (called apoptotic bodies).

The term "antibody" herein is used in the broadest sense and specifically covers monoclonal antibodies, polyclonal antibodies, multispecific antibodies {e.g. bispecific antibodies) formed from at least two intact antibodies, and antibody fragments so long as they exhibit the desired biological activity.

In a preferred embodiment, the antibody useful for the treatment of CFS is a B-cell depleting CD20-binding antibody fragment.

"CD20-binding antibody fragments" comprise a portion of an intact antibody which comprises the antigen binding region thereof. Examples of antibody fragments include Fab, Fab', F(ab')2, and Fv fragments; diabodies; linear antibodies; single-chain antibody molecules; and multispecific antibodies formed from antibody fragments. For the purposes herein, an "intact antibody" is one comprising heavy and light variable domains as well as an Fc region.

Moreover, it is assumed that other B-cell depleting agents, in particular, anti-CD22 antibodies, like Epratuzumab or anti-CD19 humanized antibodies, like MDX-1342, can be used for the treatment of CFS.

The terms "rituximab" or "RITUXAN®" or "mabthera" herein refer to the genetically engineered chimeric murine/human monoclonal antibody directed against the CD20 antigen and designated "C2B8" in US Patent No. 5,736,137, expressly incorporated herein by reference, including fragments thereof which retain the ability to bind CD20. Purely for the purposes herein and unless indicated otherwise, "humanized 2H7" refers to a humanized antibody that binds human CD20, or an antigen-binding fragment thereof, wherein the antibody is effective to deplete primate B cells in vivo.

The expression "effective amount" of the B-cell depleting agent or antagonist, in particular of the anti-CD20 antibody or CD20-binding antibody fragment thereof, refers to an amount of the B-cell depleting agent or antagonist which is effective for treating CFS. For example, the anti-CD20 antibody for the treatment of chronic fatigue syndrome/myalgic encephalomyelitis is administered in the range of 10 mg to 5000 mg per dosage. For example, the dosage may be in the range of from 100 to 1000 mg/m2, in particular, 500 mg/m2 as a single infusion for Rituximab. Typically, the dosage for Methotrexate is in the range of 5 mg to 30 mg per week.

In one preferred embodiment, the B-cell depleting agent is a chemical entity, e.g. a small molecule. A variety of B-cell depleting agents are known in the art for example known B-cell depleting agents are BAFF-antagonists. Furthermore, known B-cell depleting agents include antagonists of BR3, agonists of alpha-4-integrins etc. For example, Methotrexate is an analogue of folic acid displaying B-cell depleting activity. Other useful B-cell depleting agents are small modular immunopharmaceuticals (SMIP) against CD20. For example, SMIP acting as B-cell depleting agents are TRU-015 or SBI-087 of Trubion Pharmaceuticals. Also, SMIP can be single chain polypeptides, smaller than antibodies, having a potent B-cell depletion activity.

In a preferred embodiment, a combination of an anti CD20 antibody and representing a biological entity of a B-cell depleting agent and Methotrexat, representing a chemical entity of a B-cell depleting agent, are used for treating chronic fatigue syndrome of myalgic encephalomyelitis. Administration of these entities may be effected simultaneously, separately or sequentially. For example, in a first regimen either the antibody or Methotrexat is administered to the subject while in a second regimen the other agent is administered.

The composition comprising the B-cell depleting agent, the antagonist, in particular, the anti CD20 antibody or the CD20-binding antibody fragment thereof, will be formulated, dosed, and administered in a fashion consistent with good medical practice. Factors for consideration in this context include the stage of the particular disease or disorder being treated, the particular mammal being treated, the clinical condition of the individual subject, the site of delivery of the agent, the method of administration, the scheduling of administration, and other factors known to medical practitioners. The effective amount of the B-cell depleting agent, like an antibody or antibody fragment to be administered will be governed by such considerations. As a general proposition, the effective amount of the antagonist administered parenterally per dose will be in the range of about 20mg/m2 to about 10,000mg/m2 of subject body, by one or more dosages. Exemplary dosage regimens for intact antibodies include 375 mg/m2 weekly x 4; IOOO mg x 2 (e.g. on days 1 and 15); or 1 gram x 3. The antibody for the administration to a subject in a single therapeutically effective dosage of said antibody is of 50 to 2000 mg/m2 or multiple of therapeutically effective dosages of said antibody or CD20-binding antibody fragment thereof of 50 to 2000 mg/m2. As noted above, however, these suggested amounts of antibody are subject to a great deal of therapeutic discretion. The key factor in selecting an appropriate dose and scheduling is the result obtained, as indicated above. The B-cell depleting agent antagonist, like the antibody, is administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated. In addition, the B-cell depleting agent antagonist, like the antibody may suitably be administered by pulse infusion, e.g., with declining doses of the antagonist. Preferably the dosing is given by intravenous injections.

In another embodiment, the combination is a combination of a NO donor and a B-cell depleting agent wherein the said B-cell depleting agent is Methotrexate.

### Pharmaceutical Formulations

Therapeutic formulations of the NO donor and, optionally, the B-cell depleting agents, like antibodies or other antagonists used in accordance with the present invention are prepared for storage by mixing an antibody or a fragment thereof having the desired degree of purity with optional pharmaceutically acceptable carriers, excipients or stabilizers {Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl parabene; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Exemplary anti-CD20 antibody formulations which may form the bases of the compositions according to the present invention are described in WO98/56418, expressly incorporated herein by reference. This publication describes a liquid multidose formulation comprising 40 mg/mL rituximab, 25 mM acetate, 150 mM trehalose, 0.9% benzyl alcohol, 0.02% polysorbate 20 at pH 5.0 that has a minimum shelf life of two years storage at 2-8°C. Another anti-CD20 formulation of interest comprises 10mg/mL rituximab in 9.0 mg/mL sodium chloride, 7.35 mg/mL sodium citrate dihydrate, 0.7mg/mL polysorbate 80, and Sterile Water for Injection, pH 6.5. Lyophilized formulations adapted for subcutaneous administration are described in US Pat No. 6,267,958 (Andya et ah). Such lyophilized formulations may be reconstituted with a suitable diluent to a high protein concentration and the reconstituted formulation may be administered subcutaneously to the mammal to be treated herein. Crystalized forms of the antibody or antagonist are also contemplated. See, for example, US 2002/0136719Al.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980). Sustained-release preparations may be prepared. Suitable examples of sustained- release preparations include semipermeable matrices of solid hydrophobic polymers containing the antagonist, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), non-degradable ethylene- vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydiOxybutyric acid. The formulations to be used for in vivo administration must be sterile. This is readily accomplished by filtration through sterile filtration membranes.

Moreover, the present invention relates to a kit comprising
a) a first component which comprises a nitric oxide donor as defined herein, and
b) a second component which comprises a B-cell depleting agent as defined herein.

It is preferred, that this kit is for use in the treatment of CFS.

Finally, the present invention relates to a method for treating chronic fatigue syndrome comprising the step of administering a NO donor as defined herein to a subject afflicted with CFS.

It is preferred that the method for treating CFS with the NO donor comprises the administration of a suboptimal or low dosage at the beginning. In particular, the starting dosage may be reduced to avoid any undesired side effects. Over the time, the dosage may be increased to dosages as administered e.g. in angina pectoris. That is, the dosage is for example in the range of from 30 to 120 mg/day as it is the case for Imdur®.

Furthermore, the dosage may be an immediate release or sustained dosage depending on the way of administration.

The administration may be systemically or locally via the enteral or parenteral way. For example, topical administration may be affected, e.g. by a plaster for sustained release alternatively, the NO donor may be administered by way of inhalation or by other mucosal ways.

In a preferred embodiment, the method of treatment comprises administration of the NO donor in combination with a B-cell depleting agent. That is, a combined treatment with the NO donor and the B-cell depleting agent as defined herein is particularly preferred.

The both components may be administered simultaneously, separately or sequentially to said subject suffering from CFS. For example, the NO donor may be administered by inhalation while the B-cell depleting agent is administered by the way of infusion. Furthermore, while the NO donor may be administered on a daily basis, the B-cell depleting agent may be administered initially in once a week over two weeks and, thereafter, in a free determined time of schedule, e.g. every second or every third month.

In particular, the NO donor may be administered during the initial 4 to 12 weeks, like during the first 4 to 8 weeks of treatment, e.g. the initial 4, 5, 6, 7, 8, 9, 10, 11, 12 weeks to allow immediate relief of the symptoms of CFS while the administration at the B-cell depleting agent as defined herein allows relief to the symptoms of the subject suffering from CFS over a long time period due to the delayed effectiveness thereof.

In the following, the invention will be described further by a way of examples without limiting the same thereto.

### Examples

### Index observation

The patient was a 48 years old woman with previous thyroiditis. She had ME/CFS following mononucleosis in 1997 (33 years of age). She had a Hodgkin lymphoma stage II-A in 2003, and experienced in 2004 a transient response of her ME/CFS symptoms after chemotherapy for lymphoma relapse which has been described [Fluge O, Mella O (2009) BMC Neurol 9:28], and which later led to a pilot case series using Rituximab, also with a major but transient responses of ME/CFS symptoms for this patient. After high-dose chemotherapy with autologous stem cell support, she has been relapse-free from lymphoma since 2006. In autumn 2012, she was admitted to a local hospital with a transient ischemic attach with paresis in left side of 30 minutes duration. She had previously received radiotherapy to the mediastinum and neck as treatment for limited-stage Hodgkin lymphoma, and due to a reduced left ventricular function, her cardiologist started oral isosorbide mononitrate (Imdur®) 30 mg which she received for five days before cessation of the therapy. She had headache and some dizziness the first days, but also noticed an initial response of her ME/CFS symptoms starting within the first 24 hours. She reported a clear improvement on most ME/CFS symptoms during the next days, which she described to be remarkably alike the response she had previously experienced from 6-7 weeks after Rituximab infusion (as a pilot patient in 2007). After Imdur® treatment was stopped on day five, the symptom improvement vanished during the following two days, consistent with a direct symptomatic effect on her ME/CFS disease.

Due to the symptoms of ME/CFS seemingly from several different organ systems, a widespread biological system with many physiological functions may be affected. Taking into account the normal functions of NO, and the observed clinical improvement after Imdur® treatment, we hypothesized that NO system may be dysregulated in ME/CFS.

After discussions with the chairman of the Regional Ethical Committee, we had approval to treat ME/CFS patients that were well-known to us, with Imdur® using low doses (15 mg) initially and then slowly increasing the doses and up to 120mg/day registering subjective symptom changes as compared to baseline, in order to gain experience to draft a protocol in a planned open-label phase-II study.

All six patients were diagnosed with ME/CFS according to Canadian criteria [Carruthers BM, et al., (2003) J Chronic Fatigue Syndr 11:7-36.] and had performed standard examinations to exclude other disease explaining their condition. Three patients had previously received Rituximab with a clinical response, but then with a later full or partial relapse of ME/CFS symptoms. One patient with very severe ME/CFS had no response to previous Rituximab treatment, and one patient with a post Giardia lamblia ME/CFS had not been given Rituximab previously.

These patients had a moderate response with improvement of their CFS/ME symptoms, lasting for 3-4 months.

In this preliminary case series, data are presented indicating that the NO system is compromised in at least a subgroup of ME/CFS patients, with symptomatic clinical improvements after treatment using isosorbide mononitrate as a NO donor. It seems that the endothelium could be affected directly or indirectly by the putative autoimmune process. Endothelial dysfunction has previously been described in ME/CFS patients, with reduced flow-mediated vasodilation and reduced post-occlusive reactive hyperemia as compared to age- and sex-matched controls (Newton DJ et al., 2012, Int J Cardiol 154:335-336). Endothelial dysfunction is also a feature of some established autoimmune diseases. In addition, it has been indicated that ME/CFS patients have an increased risk of cardiovascular disease (Maes M, Twisk FN (2009), Neuro Endocrinol Lett 30:677-693).

Nitric oxide (NO) is a neurotransmitter with impact on cognitive function, and with important effects on vasodilation thus regulating blood flow, also regulating smooth muscle tone in gastrointestinal and urogenital tracts, affecting platelet aggregation and cardiac contractility, and also skeletal muscle. NO has important functions in adequate recovery sleep, and affects sensory hypersensitivity through voltage-gated potassium channels. Thus, a compromised NO system may add to many characteristic symptoms in ME/CFS patients such as cognitive disturbances, sleep problems, and hypersensitivity to stimuli such as light, noise, smell, touch, and pain. Also, disturbance in NO metabolism may explain the lower anaerobic threshold seen in ME/CFS patients, and the inhibited mitochondrial function. Several studies have shown increased lactate in the cerebrospinal fluid in ME/CFS patients (Shungu DC et al., 2012, NMR Biomed 25:1073-1087) and a strong correlation between brainstem grey matter volume and pulse pressure suggested an impaired cerebrovascular autoregulation, findings that may be ascribed to dysregulated nitric oxide. Nitric oxide also impacts on immune cells.

The NO/ONOO- pathway has been previously suggested as a pathogenetic mechanism in ME/CFS, and explained by Nitric Oxide Synthase (NOS) partial uncoupling with generation of superoxide reacting with NO and subsequent peroxynitrite causing cellular damage (Pall ML, 2000, Med. Hypotheses 54:115-125; Pall ML, 2007, Med. Hypotheses 69:821-825), and followed by the recommendation that ME/CFS patients should aim to reduce their NO levels (e.g. dietary efforts, antioxidants). This conclusion is not in line with the observed clinical effects of this case series. Oxidative stress is increased in ME/CFS patients, with oxidative stress induced damage to lipids and proteins which may be indicative of early proatherogenic processes (Tomic S, et al., 2012, Arch Med Sci 8:886-891). A model for ME/CFS has been proposed in which initial infection and immune activation, driven by oxidative and nitrosative stress with damage to self-epitopes and activation of autoreactive T-cells, and with reduced mitochondrial function and subsequent ATP deficit are involved.

The case series presented here, with a clear symptomatic relief in the first months of treatment from isosorbide mononitrate in four out of six patients suggests that a compromised NO system may be part of pathogenetic mechanisms in ME/CFS. Inducible Nitric Oxide Synthase (iNOS) in peripheral blood mononuclear cells of ME/CFS patients was shown to be higher than in healthy controls (Maes M et al., 2007, Neuro Endocrinol Lett 28:463-469). After a physical exercise test female ME/CFS patients had a higher increase NO metabolites (measured as nitrates) than a group of matched control subjects. Thus, a putative compromised NO system in ME/CFS patients does not seem to be caused by inherent deficits in components of the NO synthesis in general. Also, the responses in ME/CFS patients seen after Rituximab treatment suggests that the mechanisms for symptom maintenance are fully or partly reversible, although transiently, after B-cell depletion.

The basal levels of NO are derived from constitutive synthesis in endothelial cells (eNOS) and in nerve cells (nNOS), while the NO production can increase 100-fold by activity of inducible NOS activity (iNOS) in immune cells e.g. during infection. Taking into account the observed endothelial dysfunction in ME/CFS patients (Newton DJ et al., 2012, Int J Cardiol 154:335-336) with reduced flow-mediated vasodilation, but with intact response to sublingual glycerylnitrate, it is tempting to speculate that at least a subgroup of ME/CFS patients have a low basal NO synthesis or increased breakdown specifically in endothelial cells, and with reduced diffusion of the short-lived NO gas to tissues as one mechanism for symptom maintenance. Accordingly, some patients describe improvement of their ME/CFS symptoms such as fatigue and cognitive dysfunction during ongoing infections, which could be compatible with a raised NO level due to iNOS activity.

An alternative strategy is further investigated using L-Arginine and L-Citrulline in relatively high doses. Treatment with the semi-essential amino acid L-Arginine has being investigated (Boger 2008, Curr Opin Clin Nutr Metab Care 11: 55-61. L-Arginine supplementation has been shown to reduce endothelial dysfunction in other conditions, such as hypertension (Jabecka et al, 2012, Eur Rev Med Pharmacol Sci 16:1665-1674, and peripheral arteriosclerosis, and also to reduce the incidence of pre-eclampsia (Vadillo-Ortega et al, 2011, BMJ 342:d2901.

### Observations with the pilot patients

- Follow-up for more than 3 months.
- Pure symptomatic effect of isosorbide mononitrate. If exaggerating to much (exercise...) some ME symptoms recur.
- Two patients forgot to take the dose one day, with markedly symptoms same afternoon.
- Noctural variation in one (ME symptoms in morning before drug intake, clear improvement during afternoon and evening).
- One with very severe ME, clear effect on all symptoms except extreme sensitivity to light.
- Two patients with no effect after at least two weeks treatment with isosorbide mononitrate. Four out of six had a clear symptomatic effect after using the drug more than 2 weeks.

Taken together, the data from studies on B-cell depletion using the anti-CD20 antibody Rituximab in ME/CFS, with the observed kinetics for responses and relapses, suggest that ME/CFS is a form of autoimmune disease in a subgroup of patients. This preliminary case series showing symptomatic relief from isosorbide mononitrate, together with a known endothelial dysfunction, suggests that basal endothelial NO synthesis is disturbed in ME/CFS patients. Whether this endothelial dysfunction is an indirect feature in line with that observed in other rheumatic autoimmune diseases, or represents a direct endothelial immune target in ME/CFS, remains to be elucidated.

## Claims

1. A nitric oxide donor for use in the treatment of chronic fatigue syndrome.

2. The nitric oxide donor for use according to claim 1 which is an organic nitric oxide donor.

3. The nitric oxide donor for use according to claim 1 or 2 which is a small molecule.

4. The nitric oxide donor for use according to any one of claims 1 to 3 which is a compound selected from an organic nitrite, an organic nitrate, a nitrite ester of a polyol, a nitrate ester of a polyol molsidomine and its metabolites, a diazeniumdiolate, a S-nitrosothiol, an iron-sulphur nitrosyl, sodium nitrite, ethylene glycol dinitrate, isopropyl nitrate, amyl nitrite, amyl nitrate, ethyl nitrite, butyl nitrite, isobutyl nitrite, octyl nitrite, glyceryl-1-monoitrate, glyceryl-1 ,2-dinitrate, glyceryl-1,3-dinitrate, nitroglycerin, butane-1,2,4-triol-trinitrate, erythrityl tetranitrate, pentaerythrityl tetranitrate, sodium nitroprusside, clonitrate, erythrityl tetranitrate, isosorbide mononitrate, isosorbide dinitrate, mannitol hexanitrate, mesoionic oxatriazole, pentaerythritol tetranitrate, penetrinitol, triethanolamine trinitrate, trolnitrate phosphate (triethanolamine trinitrate diphosphate), propatylnitrate, nitrite esters of sugars, nitrate esters of sugars, sodium nitroprusside, nicorandil, apresoline, diazoxide, hydralazine, hydrochlorothiazide, minoxidil, pentaerythritol, tolazoline, scoparone (6,7-dimethoxycoumarin) sinitrodil, sildenafil, vardenafil, tadalafil, 4-Ethyl-2-[(Z)-hydroxyiminol]-5-nitro-3(E)-hexeneamide, L-arginine and pharmaceutically acceptable salts, derivatives and isomers thereof, in particular, wherein said nitric oxide donor is selected from glyceroltrinitrate, L-arginine, isosorbide mononitrate.

5. The nitric oxide donor for use according to any one of the preceding claims, wherein said nitric oxide donor is adapted for systemic administration.

6. The nitric oxide donor for use according to any one of the preceding claims adapted for the administration to a subject in a single therapeutically effective daily dosage thereof or multiple of therapeutically effective daily dosages thereof.

7. Pharmaceutical composition comprising a nitric oxide donor as defined in any one of claims 1 to 6 and a pharmaceutically acceptable diluent, excipient or carrier for use in the treatment of chronic fatigue syndrome.

8. A composition containing a combination of a nitric oxide donor as defined in any one of claims 1 to 6 and a B-cell depleting agent.

9. The composition of claim 8 in form of a pharmaceutical composition for use in the treatment of chronic fatigue syndrome, in particular, for use in the treatment of chronic fatigue syndrome wherein the combination is administered simultaneously, separately or sequentially.

10. The pharmaceutical composition of a nitric oxide donor and a B-cell depleting agent of any one of claims 8 to 9, wherein the nitric oxide donor is administered in a pharmaceutically effective dosage over a time range of the initial 4 to 12 weeks of treatment.

11. The combination of a nitric oxide donor and a B-cell depleting agent according to any one of claims 8 to 10, wherein the B-cell depleting agent is adapted for administration of one or two infusions twice within two weeks.

12. The combination of a nitric oxide donor and a B-cell depleting agent according to any one of claims 8 to 11, wherein the B-cell depleting agent is a B-cell depleting anti-CD20 antibody or CD20 binding antibody fragment thereof, preferably, a monoclonal antibody or CD20 binding antibody fragment thereof, like a humanized antibody or antibody fragment thereof or wherein the B-cell depleting agent is methotrexate.

13. A kit comprising
a) a first component which comprises a nitric oxide donor as defined in any one of claims 1 to 6 and
b) a second component which comprises a B-cell depleting agent as defined in any one of claims 8 to 12.

14. The kit according to claim 13 for use in the treatment of chronic fatigue syndrome.

15. A method for treating chronic fatigue syndrome comprising the step of administering a nitric oxide donor as defined in any one of claims 1 to 6 to a subject afflicted therewith, in particular, a method for treating chronic fatigue syndrome comprising the step of administering daily within the initial 4 to 12 weeks of a nitric oxide donor as defined in any one of claims 1 to 6, preferably whereby a combination of the nitric oxide donor as defined in any one of claims 1 to 6 with a B-cell depleting agent is administered simultaneously, separately or sequentially to a subject suffering from chronic fatigue syndrome.
